Europäisches Patentamt

European Patent Office

Office européen des brevets

(11) Veröffentlichungsnummer : **0 081 041**
B1

(12) **EUROPÄISCHE PATENTSCHRIFT**

(45) Veröffentlichungstag der Patentschrift :
02.01.86

(51) Int. Cl.⁴ : **C 07 C  5/03**, C 07 C  5/09

(21) Anmeldenummer : 82108138.7

(22) Anmeldetag : 03.09.82

(54) Verfahren zur selektiven Hydrierung von mehrfach ungesättigten Kohlenwasserstoffen in Kohlenwasserstoff-Gemischen.

(30) Priorität : 04.11.81 DE 3143647

(43) Veröffentlichungstag der Anmeldung :
15.06.83 Patentblatt 83/24

(45) Bekanntmachung des Hinweises auf die Patenterteilung : 02.01.86 Patentblatt 86/01

(84) Benannte Vertragsstaaten :
AT BE DE FR GB IT NL SE

(56) Entgegenhaltungen :
DE-A- 1 568 262
DE-A- 1 926 503
FR-A- 2 438 084
US-A- 4 251 674

(73) Patentinhaber : **HÜLS AKTIENGESELLSCHAFT**
**Patentabteilung / PB 15 - Postfach 13 20**
**D-4370 Marl 1 (DE)**

(72) Erfinder : **Obenaus, Fritz, Dr.**
**Jupiterweg 60**
**D-4370 Marl (DE)**
Erfinder : **Nierlich, Franz, Dr.**
**Vikariestrasse 16**
**D-4370 Marl (DE)**
Erfinder : **Reitemeyer, Otto, Dr.**
**Stargarder Strasse 7**
**D-4370 Marl (DE)**
Erfinder : **Scholz, Bernhard, Dr.**
**Kerkenkamp 4**
**D-4370 Marl (DE)**

## Beschreibung

Die Erfindung betrifft ein Verfahren zur selektiven Hydrierung von Kohlenwasserstoffen (KW) mit konjugierten und/oder kumulierten Doppelbindungen und/oder acetylenischen Dreifachbindungen in Monoen-haltigen Kohlenwasserstoff-Gemischen mit mindestens drei C-Atomen in flüssiger Phase an fest angeordneten Katalysatoren. Solche KW-Gemische entstehen z. B. bei der Aufarbeitung von Mineralöl.

Vor der Weiterverarbeitung derartiger KW-Gemische ist es oft notwendig, diese von mehrfach ungesättigten und acetylenischen Verbindungen zu befreien. Zweckmäßig ist die Hydrierung dieser Verbindungen. Die Erfindung bezweckt, die Hydrierung selektiv zu machen, damit möglichst keine Verluste an Monoenen durch Bildung gesättigter Kohlenwasserstoffe und gegebenenfalls durch Umlagerung zu unerwünschten ungesättigten Isomeren entstehen.

Derartige Hydrierungen können mittels bekannter fester Katalysatoren durchgeführt werden. Besonders geeignet sind Metalle aus der achten Gruppe und der ersten Nebengruppe des Periodensystems, die z. B. auf Bimsstein, Tone oder Silikate als Trägermaterial aufgebracht werden. Es sind mehrere Verfahren bekannt, bei denen die Selektivität durch ausgewählte Reaktionsbedingungen oder durch Modifizierung des Katalysators erhöht wird.

Günstig ist die « Kalthydrierung » bei relativ niedriger Temperatur (DE-PS-1 568 542), die Hydrierung in der Flüssigphase mit gelöstem Wasserstoff (DE-PS-1 210 795) und die Hydrierung von Dienen zu Monoenen an Palladium-Katalysatoren in Gegenwart von Ammoniak (BE-802 721).

Weiter ist vorgeschlagen worden, die Katalysatoren mit Schwefelverbindungen zu modifizieren. So kann man beispielsweise durch Behandeln mit Thioethern Katalysatoren erhalten, die selektiv auf die Hydrierung von Acetylen wirken (FR-1 240 175). Mit Schwefelwasserstoff dotierte Katalysatoren eignen sich für die selektive Hydrierung von Butadien ; sie katalysieren jedoch gleichzeitig die Isomerisierung von z. B. Buten-1 zu Buten-2 (FR-2 355 792).

Auch der Zusatz von Kohlenmonoxid in kleinen Mengen katalysiert in Gegenwart von Wasserstoff an Palladium-Katalysatoren die Isomerisierung von Buten-1 zu Buten-2 (FR-A-2 438 084).

In DE-OS-1 926 503 wird die selektive Hydrierung von mehrfach ungesättigten KW mit mindestens vier C-Atomen in Anwesenheit von mono-olefinischen KW zu einfach ungesättigten Verbindungen an einem Palladium- oder Nickel-Katalysator beschrieben, wobei dem flüssigen Reaktionsgemisch neben Wasserstoff noch 1 bis 50 Molprozent Kohlenmonoxid, bezogen auf den Wasserstoff, zugesetzt werden. Dadurch soll die Wanderung von Doppelbindungen (Isomerisierung) verhindert werden.

Bei dieser selektiven Hydrierung liegt jedoch stets eine Gasphase neben dem flüssigen Reaktionsgemisch vor, da die zugegebenen Wasserstoffmengen erheblich größer sind als der Löslichkeit von Wasserstoff in dem flüssigen Reaktionsgemisch unter den vorliegenden Druck- und Temperaturbedingungen entspricht.

Wie weiter ausdrücklich festgestellt wird, ist es notwendig, mehr als die stöchiometrische Menge Wasserstoff, die für die Hydrierung der mehrfach ungesättigten KW in die entsprechenden mono-olefinischen KW erforderlich ist, zu verwenden ; üblicherweise werden 1 bis 20 000 Mol Wasserstoff zugegeben. Kohlenmonoxid in einer Menge von weniger als 1 Molprozent, bezogen auf Wasserstoff, ist nicht zweckmäßig, da bei einer kleineren Menge Kohlenmonoxid während der Hydrierung Doppelbindungen wandern können. Die in den Beispielen genannten Kohlenmonoxid-Mengen entsprechen 40 000 bis 500 000 Massen-ppm, bezogen auf die Kohlenwasserstoff-Menge.

Bei dem beschriebenen Verfahren werden bevorzugt 10 Molprozent Kohlenmonoxid, bezogen auf Wasserstoff, zugegeben. Trotzdem wird die gestellte Aufgabe (Verhindern des Wanderns von Doppelbindungen) nicht gelöst. Gemäß Figur 2 nimmt mit fortschreitender Hydrierung von Butadien der Gehalt an Buten-1 um etwa 10 % ab, obwohl bei selektiver Hydrierung ohne Isomerisierung ein zunehmender Gehalt an Buten-1 zu erwarten ist.

Die bekannten Verfahren sind unbefriedigend, weil ein gewisser Anteil der ungesättigten Kohlenwasserstoffe vollständig hydriert wird und in vielen Fällen eine Isomerisierung nicht verhindert werden kann. Im Falle der Hydrierung von Butadien in Gemischen aus $C_4$-Kohlenwasserstoffen wird beispielsweise Buten-1 zu Buten-2 isomerisiert.

Damit stellt sich die Aufgabe, ein einfaches Verfahren zur selektiven Hydrierung von Kohlenwasserstoffen mit konjugierten und/oder kumulierten Doppelbindungen und/oder acetylenischen Dreifachbindungen in Monoen-haltigen KW-Gemischen mit mindestens drei C-Atomen in flüssiger Phase an fest angeordneten Palladium-Katalysatoren auf inertem Träger zu entwickeln, bei dem die hydrierten Verbindungen in einfach ungesättigte Verbindungen übergehen und als solche erhalten bleiben, und bei dem außerdem keine Isomerisierung eintritt.

Die Aufgabe wird erfindungsgemäß gelöst durch ein Verfahren mit folgenden kennzeichnenden Merkmalen :

Zugeben von Wasserstoff zu dem zu hydrierenden Kohlenwasserstoff-Gemisch in feinverteilter Form und in solchen Mengenverhältnissen, bei denen stets eine homogene Flüssigphase vor Eintritt des Kohlenwasserstoff-Gemisches in die Hydrierzone erhalten wird, und die dem Einfachen bis Doppelten der stöchiometrischen Menge zum Hydrieren der mehrfach ungesättigten sowie der acetylenischen Verbindungen zu den entsprechenden Monoenen entsprechen,

0 081 041

Zugeben von Kohlenmonoxid zu dem zu hydrierenden Kohlenwasserstoff-Gemisch in feinverteilter Form, wobei stets eine homogene Flüssigphase vor Eintritt des Kohlenwasserstoff-Gemisches in die Hydrierzone erhalten wird, und der Anteil von Kohlenmonoxid mindestens 0,05 Massen-ppm beträgt — bezogen auf die Masse des Kohlenwasserstoff-Gemisches —,

Überleiten des so gebildeten Reaktionsgemisches in flüssiger Phase über einen fest angeordneten Katalysator mit 0,01 bis 3 Massen-% Palladium, bezogen auf die Masse des Trägers.

Am Katalysator liegt eine homogene Flüssigphase vor, keine Gasphase, d. h. Wasserstoff ($H_2$) und Kohlenmonoxid (CO) sind in dem zu hydrierenden KW-Gemisch vollständig gelöst.

Die stöchiometrische $H_2$-Menge ist diejenige, die zur Umwandlung der mehrfach ungesättigten und der acetylenischen Verbindungen in ihre Monoene rechnerisch notwendig ist. Sie läßt sich aus der Zusammensetzung des zu hydrierenden KW-Gemisches berechnen.

Die auf die Menge des KW-Gemisches zu beziehende CO-Mindestmenge wird experimentell ermittelt, indem man bei Umsetzung einer mindestens stöchiometrischen $H_2$-Menge die CO-Zugabe stufenweise erhöht und jeweils nach der Hydrierung die Konzentration der gewünschten Monoene mißt. Die CO-Mindestmenge ist diejenige, bei der unter den gewählten hydrierbedingungen die Konzentration der gewünschten Monoene im hydrierten KW-Gemisch ihr Maximum erreicht hat. Die gewünschten Monoene können bei einem Gemisch aus $C_3$-Kohlenwasserstoffen das Propen, bei Kohlenwasserstoffen mit vier und mehr C-Atomen eines der Monoen-Isomeren oder deren Summe sein.

Beim Einsatz einer konstanten $H_2$-Menge nimmt die CO-Mindestmenge mit dem Palladiumgehalt des Katalysators und mit der Hydriertemperatur zu. Wird aus praktischen Gründen die $H_2$-Menge erhöht, ist auch die CO-Menge zu erhöhen.

Das Überschreiten der CO-Mindestmenge ändert das Ergebnis der selektiven Hydrierung nicht. Der obere Grenzwert der CO-Menge wird erreicht, wenn sich das CO-Gas nicht mehr vollständig im zu hydrierenden KW-Gemisch löst, wenn also am Katalysator eine heterogene Mischphase aus Gas und Flüssigkeit entsteht.

Die CO-Menge, bezogen auf die Masse des KW-Gemisches, beträgt mindestens 0,05 Massen-ppm. Dosierungen oberhalb 20 Massen-ppm verbessern erfahrungsgemäß unter den übrigen gewählten Bedingungen die erzielbaren Ergebnisse nicht mehr. Das erfindungsgemäße Verfahren ist an keinen bestimmten Palladium-Katalysator gebunden. Der Katalysator enthält 0,01 bis 3 Massen-% Palladium, vorzugsweise 0,1 bis 2 Massen-%.

Die Katalysatorträger sollen inert sein, d. h. sie sollen die selektive Hydrierung nicht beeinträchtigen. Zu solchen Trägern gehören z. B. Aluminiumoxid ($Al_2O_3$), Silicagel und Aktivkohle.

Einen nachgeordneten Einfluß auf das erfindungsgemäße Verfahren haben die übrigen Parameter der Hydrierung, nämlich Reaktionstemperatur, Reaktionsdruck, Konzentration der zu hydrierenden Komponenten im KW-Gemisch, Art der Zumischung von $H_2$ und CO zum KW-Gemisch und Durchsatz an KW-Gemisch.

Die Reaktionstemperatur ist für das erfindungsgemäße Verfahren nur insofern zu beachten, als die CO-Mindestmenge mit der Temperatur ansteigt. Wegen der großen Reaktionsgeschwindigkeit ist die selektive Hydrierung auch bei tiefen Temperaturen möglich. Die untere Grenze der Reaktionstemperatur wird vorrangig durch praktische Gründe bestimmt, die für den Gegenstand der Erfindung unwesentlich sind.

Bei wasserhaltigen KW-Gemischen wird die untere Reaktionstemperatur bei etwa 0 °C liegen. Die obere Grenze der Reaktionstemperatur wird durch die kritischen Daten des KW-Gemisches vor und nach der Hydrierung bestimmt ; diese sind beispielsweise für Propen 91,9 °C und 4,5 MPa. Damit liegt die Obergrenze der Reaktionstemperatur beispielsweise bei Anwesenheit von Propen etwa bei 90 °C. Soll die Hydrierung bei hoher Temperatur verlaufen, sind die Apparate für den entsprechenden Druck auszulegen. Praktisch wird eine Temperatur zwischen 10 °C und 75 °C bevorzugt.

Der Reaktionsdruck hat nur mittelbaren Einfluß auf das erfindungsgemäße Verfahren. Er muß hinreichend groß sein, damit die Flüssigphase am Katalysator erhalten bleibt. Der Druck kann heraufgesetzt werden, wenn die Mengen an zu lösendem $H_2$ und CO erhöht werden sollen. Im allgemeinen wird ein Reaktionsdruck von etwa 1,5 MPa angewendet ; 6 MPa werden nur selten überschritten. Man kann durch Wahl eines geeigneten Reaktionsdruckes und einer geeigneten Reaktionstemperatur für alle Gemisch-Zusammensetzungen die Flüssigphase am Katalysator erhalten.

Falls die benötigte $H_2$-Konzentration zu groß ist, um bei den gewünschten Reaktionsbedingungen im KW-Gemisch vollständig in Lösung zu gehen, kann man das KW-Gemisch zwei- oder mehrstufig hydrieren oder das hydrierte KW-Gemisch teilweise im Kreislauf fahren.

Einige Beispiele für als Einsatzstoff geeignete KW-Gemische sind in den Tabellen 1 und 2 zusammengestellt.

Das zu hydrierende KW-Gemisch wird nach einem der bekannten Verfahren mit feinverteiltem $H_2$ und CO versetzt, damit sich die Gase schneller lösen.

Der Durchsatz an zu hydrierendem KW-Gemisch durch den Katalysator liegt in dem für Hydrierreaktionen üblichen Bereich von 5 bis 300 Liter KW-Gemisch pro Liter Katalysatorvolumen und Stunde.

Die Zusammensetzung des KW-Gemisches vor und nach der Hydrierung wird vorzugsweise gaschromatographisch bestimmt. Aus den Konzentrationsänderungen der Komponenten werden Umsatz und Isomerisierung berechnet.

3

0 081 041

Überraschenderweise ist es nach dem erfindungsgemäßen Verfahren möglich, die mehrfach ungesättigten und die acetylenischen Verbindungen bis zu den entsprechenden Monoenen quantitativ zu hydrieren, und zwar bereits unter Einsatz der stöchiometrischen $H_2$-Menge. Der Restgehalt an mehrfach ungesättigten und an acetylenischen Verbindungen im KW-Gemisch liegt nach der selektiven Hydrierung dann an der Nachweisgrenze. Die ursprünglich vorhandenen und die bei der selektiven Hydrierung entstandenen Monoene bleiben bei der selektiven Hydrierung unverändert. Dies ist besonders im Hinblick auf die Lehre in der Patentschrift FR-2 438 084 überraschend.

Das erfindungsgemäße Verfahren hat folgende Vorteile :

Die zu hydrierenden Verbindungen werden praktisch quantitativ selektiv hydriert.

Die Monoene werden nicht zu gesättigten Verbindungen hydriert ; dabei ist es gleichgültig, ob die Monoene vor der Hydrierung im KW-Gemisch bereits vorhanden waren oder durch die Hydrierung entstanden sind.

Die Hydrierung ist in einem sehr großen Konzentrationsbereich der mehrfach ungesättigten und der acetylenischen Verbindungen selektiv.

Es tritt keine nachweisbare Isomerisierung der Monoene auf ; beispielsweise wird Buten-1 nicht zu Buten-2 isomerisiert.

Der Katalysator erreicht die gewünschte Selektivität sofort bei Anwesenheit der CO-Mindestmenge.

Weder an das zu hydrierende KW-Gemisch, noch an das $H_2$- und an das CO-Gas werden besondere Reinheitsanforderungen gestellt, solange die CO-Mindestmenge und die stöchiometrische $H_2$-Menge eingehalten werden und die Nebenbestandteile keine Katalysatorgifte sind.

Im KW-Gemisch gelöstes Wasser ist nicht störend. In Gegenwart von gelöstem Wasser läßt sich die CO-Mindestmenge sogar etwas herabsetzen.

Da auch bei höherer Reaktionstemperatur die Selektivität erhalten bleibt, benötigt man für das erfindungsgemäße Verfahren keine aufwendigen Kühlvorrichtungen oder Anlagen zur Kälteerzeugung.

Da außer den gewünschten Reaktionen keine neben- oder Folgereaktionen ablaufen, treten keine zusätzlichen Wärmeeffekte auf, wodurch die Wärmeabführung vereinfacht wird.

Die Dosierung der $H_2$- und der CO-Menge kann mit automatisch arbeitenden Analyseverfahren leicht geregelt werden.

Das erfindungsgemäße Verfahren gestattet beispielsweise Buten-1 in einer für Polymerisationen geeigneten Qualität aus $C_4$-KW-Gemischen, die neben Buten-1 auch Butadien und acetylenische Verbindungen enthalten, destillativ zu gewinnen. Reste von überschüssigem $H_2$ und das erfindungsgemäß im KW-Gemisch gelöste CO stören hierbei nicht.

Die Erfindung wird an Hand der folgenden Beispiele erläutert, ohne auf diese beschränkt zu sein.

Zusammensetzung des zu hydrierenden KW-Gemisches und Hydrierbedingungen

Für die folgenden Beispiele werden KW-Gemische mit der in Tabelle 1 und Tabelle 2 angegebenen Zusammensetzung eingesetzt. Die Konzentration ist in Massen-% oder Massen-ppm angegeben und auf die Menge des KW-Gemisches bezogen. Zusätzlich ist die stöchiometrische $H_2$-Konzentration aufgeführt.

Obwohl die stöchiometrische $H_2$-Menge für die selektive Hydrierung hinreichend ist, wird im allgemeinen eine etwas größere $H_2$-Konzentration gewählt, um Schwankungen in der Konzentration der zu hydrierenden Verbindungen aufzufangen.

Nach Zugabe der in den Beispielen jeweils genannten $h_2$- und CO-Mengen, die im KW-Gemisch gelöst sind, wird das KW-Gemisch als Flüssigphase an einem fest angeordneten Palladium-Katalysator auf inertem Träger unter den angegebenen Bedingungen hydriert. Der Durchsatz wird in Liter KW-Gemisch pro Liter Katalysatorvolumen und Stunde angegeben.

Tabelle 1

Zusammensetzung der in den Beispielen A, B und 1 bis 22 eingesetzten KW-Gemische

| KW-Gemisch eingesetzt für Beispiel | Konzentration der Verbindungen | | | | |
|---|---|---|---|---|---|
| | A, B<br>1 - 5<br>21 | 6 - 8<br>9 - 12<br>18 - 20 | 13 - 17 | 22 | |
| Propan | | | | 1,2 | % |
| Propen | | | | 0,3 | % |
| Propadien | | | | 0,180 | % |
| Propin (Methyl-Acetylen) | | | | 0,620 | % |

4

(Fortsetzung)

| KW-Gemisch eingesetzt für Beispiel | A, B 1 – 5 21 | 6 – 8 9 – 12 18 – 20 | 13 – 17 | 22 | |
|---|---|---|---|---|---|
| iso-Butan | 0,028 | 0,032 | 0,040 | 0,130 | % |
| n-Butan | 21,5 | 21,6 | 22,2 | 13,0 | % |
| iso-Buten | 0,072 | 0,082 | 0,110 | – | % |
| Buten-1 | 48,4 | 48,2 | 53,9 | 37,4 | % |
| cis-Buten-2 | 14,2 | 14,1 | 8,8 | – | % |
| trans-Buten-2 | 15,6 | 15,5 | 14,7 | 26,8 | % |
| Butadien-1,3 | 0,196 | 0,514 | 0,261 | 19,5 | % |
| Butadien-1,2 | – | – | – | 0,073 | % |
| Butin-1 (Ethyl-Acetylen) | 0,0056 | 0,0056 | 0,0030 | 0,058 | % |
| Butenin (Vinyl-Acetylen) | 0,0012 | 0,0012 | 0,0025 | 0,512 | % |
| KW mit 5 und mehr C-Atomen | | | | 0,2 | % |
| Stöchiometrische $H_2$-Konzentration | 76 | 194 | 101 | 8135 | ppm |

Tabelle 2

Zusammensetzung der in den Beispielen 23 und 24 eingesetzten KW-Gemische

| KW-Gemisch eingesetzt für Beispiel | Konzentration der Verbindungen | | |
|---|---|---|---|
| | 23 | 24 | |
| Propan | 6,35 | | % |
| Propen | 93,3 | | % |
| Propadien | 0,21 | | % |
| Propin (Methyl-Acetylen) | 0,15 | | % |
| Stöchiometrische $H_2$-Konzentration | 182 | | ppm |
| Pentan | | 5,68 | % |
| Penten | | 81,9 | % |
| Isopren | | 12,4 | % |
| Stöchiometrische $H_2$-Konzentration | | 3680 | ppm |

Vergleichsbeispiel : Hydrierung ohne CO-Zusatz

Im KW-Gemisch nach Tabelle 1 wird einmal etwas mehr als die stöchiometrische $H_2$-Menge gelöst (Beispiel A), zum anderen etwa das Doppelte dieser Menge (Beispiel B). Das Gemisch (Wassergehalt < 5 ppm) wird unter folgenden Bedingungen hydriert :
Temperatur 21 °C   Durchsatz 35 Liter/Liter · Stunde
Druck 1,3 MPa   Katalysator 0,5 % Pd auf $Al_2O_3$

Dabei hat sich ergeben :

| | | KW-Gemisch enthält | | | | $\Delta$ c |
|---|---|---|---|---|---|---|
| | | Buta-dien ppm | Butin ppm | But-enin ppm | Buten -1 % abs | % rel |
| vor der Hydrierung | | 1960 | 56 | 12 | 48,4 | – |
| nach der Hydrierung | | | | | | |
| Beispiel | Konzentration H$_2$ | | | | | |
| A | 80 ppm | 650 | 6 | 2 | 47,1 | – 2,7 |
| B | 150 ppm | 43 | 1 | 1 | 44,3 | – 8,3 |

$\Delta$c ist die relative Änderung der Konzentration von Buten-1, bezogen auf dessen Konzentration vor der Hydrierung.

In beiden Fällen werden Butadien, Butin und Butenin nicht vollständig hydriert, wenngleich bei einem H$_2$-Überschuß von etwa 100 % der stöchiometrischen Menge ein größerer Teil dieser Verbindungen hydriert wird.

Die Konzentration von Buten-1 wird in beiden Fällen während der Hydrierung deutlich herabgesetzt ; ein Teil von Buten-1 wird zu Butan hydriert oder zu Buten-2 isomerisiert. Beides ist für die Gewinnung von Buten-1 ungünstig.

Beispiel 1 bis 5 : Einfluß der Reaktionstemperatur und der CO-Konzentration auf die Hydrierung

Im KW-Gemisch nach Tabelle 1 werden 85 ppm H$_2$, also etwas mehr als die stöchiometrische H$_2$-Menge (76 ppm), und die an die Hydriertemperatur angepaßte variable CO-Mindestmenge gelöst. Das KW-Gemisch (Wassergehalt < 5 ppm) wird unter folgenden Bedingungen hydriert :
Temperatur variabel   Durchsatz 35 Liter/Liter · Stunde
Druck 1,3 MPa   Katalysator 0,5 % Pd auf Al$_2$O$_3$
Dabei hat sich ergeben :

| | | | KW-Gemisch enthält | | | |
|---|---|---|---|---|---|---|
| | | | Buta-dien ppm | Butin ppm | But-enin ppm | Buten-1 % |
| vor der Hydrierung | | | 1960 | 56 | 12 | 48,4 |
| nach der Hydrierung | | | | | | |
| Beispiel | Tempe-ratur °C | Konzentration CO ppm | | | | |
| 1 | 5 | 0,4 | 2 | < 1 | < 1 | 48,5 |
| 2 | 21 | 0,6 | 3 | < 1 | < 1 | 48,5 |
| 3 | 41 | 1,8 | 1 | < 1 | < 1 | 48,5 |
| 4 | 55 | 4,2 | 2 | < 1 | < 1 | 48,5 |
| 5 | 75 | 10,0 | 1 | < 1 | < 1 | 48,4 |

Im gesamten Temperaturbereich werden Butadien, Butin und Butenin praktisch vollständig hydriert, wenn man die angegebene Konzentration an gelöstem CO einsetzt. Die CO-Mindestkonzentration steigt im untersuchten Temperaturbereich stark an.

Im Gegensatz zu den Vergleichsbeispielen nimmt die Konzentration an Buten-1 nicht ab, sondern bleibt praktisch unverändert.

Beispiel 6 bis 8 : Einfluß der CO-Konzentration auf die Hydrierselektivität bei erhöhter Reaktionstemperatur

Im KW-Gemisch nach Tabelle 1 werden 210 ppm $H_2$, also etwas mehr als die stöchiometrische $H_2$-Menge (194 ppm), und eine variable CO-Menge gelöst. Das KW-Gemisch (Wassergehalt < 5 ppm) wird unter folgenden Bedingungen hydriert :
Temperatur 55 °C   Durchsatz 35 Liter/Liter · Stunde
Druck 1,3 MPa   Katalysator 0,5 % Pd auf $Al_2O_3$
Dabei hat sich ergeben :

| | | KW-Gemisch enthält | | | |
| | | Buta-dien ppm | Butin ppm | But-enin ppm | Buten-1 % |
|---|---|---|---|---|---|
| vor der Hydrierung | | 5140 | 52 | 12 | 48,2 |
| nach der Hydrierung | | | | | |
| Beispiel | Konzentration CO ppm | | | | |
| 6 | 4,2 | 4 | < 1 | <1 | 48,4 |
| 7 | 1,8 | 100 | < 1 | <1 | 48,2 |
| 8 | 0,6 | 250 | < 1 | <1 | 47,5 |

Unter diesen Hydrierbedingungen ist die CO-Konzentration 4,2 ppm die Mindestkonzentration. Die CO-Konzentrationen 1,8 ppm und 0,6 ppm sind eindeutig kleiner als die Mindestkonzentration ; in beiden Beispielen wird Butadien unvollständig hydriert, Butin und Butenin dagegen werden vollständig hydriert. Die Konzentration von Buten-1 ist in den Beispielen 7 und 8 kleiner als in Beispiel 6.

Beispiel 9 bis 12 : Einfluß der CO-Konzentration auf die Hydrierselektivität bei niedriger Reaktionstemperatur

Im KW-Gemisch nach Tabelle 1 werden analog zu den Beispielen 6 bis 8 wiederum 210 ppm $H_2$, also etwas mehr als die stöchiometrische $H_2$-Menge (194 ppm), und eine variable CO-Menge gelöst. Das KW-Gemisch wird unter folgenden Bedingungen hydriert :
Temperatur 21 °C   Durchsatz 35 Liter/Liter · Stunde
Druck 1,3 MPa Katalysator 0,5 % Pd auf $Al_2O_3$
Dabei hat sich ergeben :

| | | KW-Gemisch enthält | | | |
| | | Buta-dien ppm | Butin ppm | But-enin ppm | Buten-1 % |
|---|---|---|---|---|---|
| vor der Hydrierung | | 5140 | 52 | 12 | 48,2 |
| nach der Hydrierung | | | | | |
| Beispiel | Konzentration CO ppm | | | | |
| 9 | 0,1 | 780 | < 1 | <1 | 47,3 |
| 10 | 0,4 | 230 | < 1 | <1 | 47,8 |
| 11 | 0,6 | 1 | < 1 | <1 | 48,3 |
| 12 | 1,8 | 2 | < 1 | <1 | 48,4 |

Unter diesen Hydrierbedingungen sind 0,6 ppm CO die Mindestkonzentration. 0,1 ppm und 0,4 ppm sind eindeutig zu klein ; in beiden Fällen wird Butadien unvollständig hydriert, und die Konzentration von Buten-1 ist nach der Hydrierung kleiner als vorher. 1,8 ppm CO bringen keinen Vorteil gegenüber der Mindestkonzentration in Beispiel 11.

Beispiel 13 bis 17 : Ermittlung der CO-Mindestmenge

Im KW-Gemisch nach Tabelle 1 werden eine variable CO-Menge und eine variable $H_2$-Menge gelöst ; letztere ist meist größer als die stöchiometrische Menge (101 ppm). Das Gemisch wird unter folgenden Bedingungen hydriert :

Temperatur 40 °C   Durchsatz 21 Liter/Liter · Stunde
Druck 1,3 MPa   Katalysator 0,5 % Pd auf Aktivkohle
Dabei hat sich ergeben :

| | | | | KW-Gemisch enthält | | | |
|---|---|---|---|---|---|---|---|
| | | | | Buta-dien ppm | Butin ppm | But-enin ppm | Buten-1 % |
| vor der Hydrierung | | | | 2610 | 30 | 25 | 53,9 |
| nach der Hydrierung | | | | | | | |
| Beispiel | Konzentration | | Ü | | | | |
| | CO ppm | $H_2$ ppm | % | | | | |
| 13 | 1,7 | 105 | 4 | 2 | < 1 | < 1 | 54,0 |
| 14 | 1,7 | 150 | 49 | 3 | < 1 | < 1 | 53,5 |
| 15 | 3,4 | 150 | 49 | 2 | < 1 | < 1 | 53,9 |
| 16 | 1,7 | 180 | 78 | 2 | < 1 | < 1 | 52,9 |
| 17 | 7,0 | 180 | 78 | 4 | < 1 | < 1 | 53,9 |

Ü ist der relative Überschuß über die stöchiometrische Menge.

Die Konzentration 1,7 ppm CO ist hinreichend, wenn ohne $H_2$-Überschuß gearbeitet wird ; sie ist dagegen zu klein, wenn der $H_2$-Überschuß 49 % beträgt. Andererseits sind 3,4 ppm CO bei 49 % $H_2$-Überschuß bereits hinreichend. Bei 78 % $H_2$-Überschuß sind 7,0 ppm CO nötig.

Beispiel 18 bis 20 : Beziehung zwischen CO-Konzentration und Reaktionsdruck

Im KW-Gemisch nach Tabelle 1 werden 205 ppm $H_2$, also etwas mehr als die stöchiometrische $H_2$-Menge (194 ppm), und 0,6 ppm CO gelöst. Das Gemisch wird unter folgenden Bedingungen hydriert :

Temperatur 21 °C   Durchsatz 35 Liter/Liter · Stunde
Druck variabel   Katalysator 0,5 % Pd auf $Al_2O_3$
Dabei hat sich ergeben :

| | | KW-Gemisch enthält | | | |
|---|---|---|---|---|---|
| | | Buta-dien ppm | Butin ppm | But-enin ppm | Buten-1 % |
| vor der Hydrierung | | 5140 | 56 | 12 | 48,2 |
| nach der Hydrierung | | | | | |
| Beispiel | Druck MPa | | | | |
| 18 | 1,0 | 2 | < 1 | < 1 | 48,5 |
| 19 | 1,5 | 1 | < 1 | < 1 | 48,4 |
| 20 | 1,8 | 3 | < 1 | < 1 | 48,5 |

Der Druck, bei dem das Gemisch hydriert wird, hat im untersuchten Bereich praktisch keinen Einfluß auf das Ergebnis der Hydrierung. Gemessen an der Konzentration von Buten-1 ist die CO-Konzentration vom Reaktionsdruck praktisch unabhängig.

Beispiel 21 : Einfluß des Wassergehaltes auf die CO-Mindestmenge

Im Gemisch nach Tabelle 1 wurden $H_2$ und CO gelöst, die Wasserkonzentration wurde variiert. Das Gemisch wurde unter folgenden Bedingungen hydriert :
Temperatur 21 °C   Durchsatz 35 Liter/Liter · Stunde
Druck 1,3 MPa   Katalysator 0,5 %Pd auf $Al_2O_3$
Bei 300 ppm Wasser erreicht man die vollständige Hydrierung von Butadien, Butin und Butenin mit einer deutlich kleineren CO-Konzentration als bei etwa 5 ppm Wasser.

| | | | KW-Gemisch enthält | | | |
|---|---|---|---|---|---|---|
| | | | Buta-dien ppm | Butin ppm | But-enin ppm | Buten-1 % |
| vor der Hydrierung | | | 1960 | 56 | 12 | 48,4 |
| nach der Hydrierung | | | | | | |
| Beispiel | Konzentration | | | | | |
| | $H_2$ ppm | CO ppm | $H_2O$ ppm | | | |
| 2 | 85 | 0,6 | <5 | 3 | < 1 | < 1 | 48,4 |
| 21 | 80 | 0,3 | 300 | 2 | < 1 | < 1 | 48,5 |

Setzt man die stöchiometrische $H_2$-Konzentration von 76 ppm ein, erhält man innerhalb der Nachweisgenauigkeit dasselbe Ergebnis.

Beispiel 22 : Selektive Hydrierung bei großer Konzentration der mehrfach ungesättigten Kohlenwasserstoffe

Das Gemisch nach Tabelle 1 mit etwa 21 % an zu hydrierenden Verbindungen benötigt die stöchiometrische Menge von 8 135 ppm $H_2$ bei der Hydrierung der mehrfach ungesättigten $C_4$-Verbindungen zu Buten und der mehrfach ungesättigten $C_3$-Verbindungen zu Propen. In diesem KW-Gemisch wird 1 ppm Co gelöst. Das Gemisch wird zuerst in einen Kreislaufreaktor gefahren. In den Kreislaufstrom werden — bezogen auf das frisch zugeführte Gemisch — 7 980 ppm $H_2$ gegeben und darin homogen gelöst. Das wasserstoffhaltige Gemisch wird im Kreislauf bei 25 °C und 1,3 MPa an einem Katalysator mit 0,5 % Pd auf $Al_2O_3$ hydriert. Das Verhältnis von rückgeführter Menge zu eingegebener Menge beträgt 49 : 1. Der Gesamtdurchsatz im Kreislaufreaktor beträgt 68 Liter/Liter · Stunde. In dem aus dem Kreislaufreaktor entnommenen Gemisch werden weitere 250 ppm $H_2$ gelöst, und das Gemisch wird bei 25 °C und 1,3 MPa mit 27 Liter/Liter · Stunde in einem Nachreaktor nachhydriert. Vor dem Nachreaktor wird kein weiteres CO zugesetzt.
Dabei hat sich ergeben :

| KW-Gemisch enthält | vor der Hydrierung | nach der Hydrierung | | |
|---|---|---|---|---|
| | | am Ausgang des Kreislauf-reaktors | am Ausgang des Nach-reaktors | |
| Butadien | 195 730 | 4 710 | 2 | ppm |
| Butin | 580 | 4 | < 1 | ppm |
| Butenin | 5 120 | 2 | < 1 | ppm |
| Propadien | 1 800 | 270 | < 1 | ppm |
| Propin | 6 200 | 8 | < 1 | ppm |

Die Buten-1-Konzentration hat von 37,4 % auf 48,6 % zugenommen, die Propen-Konzentration von 0,3 % auf 1,1 %.

Nach dem erfindungsgemäßen Verfahren wird auch ein Anteil von etwa 21 % an mehrfach ungesättigten Verbindungen selektiv hydriert.

Beispiel 23 : Selektive Hydrierung eines $C_3$-KW-Gemisches

Im KW-Gemisch nach Tabelle 2 werden 195 ppm $H_2$, also etwas mehr als die stöchiometrische $H_2$-Menge (182 ppm), und 2,5 ppm CO gelöst. Das KW-Gemisch wird unter folgenden Bedingungen hydriert :

Temperatur 45 °C   Durchsatz 40 Liter/Liter · Stunde

Druck 3,0 MPa   Katalysator 0,1 % Pd auf $Al_2O_3$

Dabei hat sich ergeben :

| | KW-Gemisch enthält | | |
| --- | --- | --- | --- |
| | Propa-dien ppm | Propin ppm | Propen % |
| vor der Hydrierung | 2 100 | 1 500 | 93,3 |
| nach der Hydrierung | 3 | < 1 | 93,6 |

Propadien und Propin sind also quantitativ zu Propen hydriert worden.

Beispiel 24 : Selektive Hydrierung eines $C_3$-KW-Gemisches

Das KW-Gemisch nach Tabelle 2 mit etwa 12 % an zu hydrierenden Verbindungen benötigt die stöchiometrische Menge von 3 680 ppm $H_2$ bei der Hydrierung der mehrfach ungesättigten $C_5$-Verbindungen zu Pentenen. In diesem Gemisch werden 1,2 ppm CO gelöst. Das KW-Gemisch wird bei 28 °C und 1,8 MPa in einen Kreislaufreaktor gefahren. In den Kreislaufstrom werden — bezogen auf das frisch zugeführte Gemisch — 3 800 ppm $H_2$ gegeben und darin homogen gelöst. Das wasserstoffhaltige Gemisch wird an einem Katalysator mit 2,0 % Pd auf $Al_2O_3$ bei einem Gesamtdurchsatz von 23 Liter/Liter · Stunde hydriert. Das Verhältnis der rückgeführten Menge zur eingegebenen Menge beträgt 26 : 1. Der Durchsatz an frisch zugeführtem Gemisch beträgt 0,9 Liter/Liter · Stunde. Ohne zusätzliche Hydrierung in einem Nachreaktor hat sich ergeben :

| | KW-Gemisch enthält | |
| --- | --- | --- |
| | Isopren ppm | Pentene % |
| vor der Hydrierung | 124 000 | 81,9 |
| nach der Hydrierung | 30 | 94,3 |

Isopren ist also quantitativ selektiv hydriert worden.

**Patentansprüche**

1. Verfahren zur selektiven Hydrierung von Kohlenwasserstoffen mit konjugierten und/oder kumulierten Doppelbindungen und/oder acetylenischen Dreifachbindungen in Monoen-haltigen Kohlenwasserstoff-Gemischen mit mindestens drei C-Atomen in flüssiger Phase an fest angeordneten Palladium-Katalysatoren auf inertem Träger, gekennzeichnet durch

Zugeben von Wasserstoff zu dem zu hydrierenden Kohlenwasserstoff-Gemisch in feinverteilter Form und in solchen Mengenverhältnissen, bei denen stets eine homogene Flüssigphase vor Eintritt des Kohlenwasserstoff-Gemisches in die Hydrierzone erhalten wird, und die dem Einfachen bis Doppelten der stöchiometrischen Menge zum Hydrieren der mehrfach ungesättigten sowie der acetylenischen Verbindungen zu den entsprechenden Monoenen entsprechen,

Zugeben von Kohlenmonoxid zu dem zu hydrierenden Kohlenwasserstoff-Gemisch in feinverteilter Form, wobei stets eine homogene Flüssigphase vor Eintritt des Kohlenwasserstoff-Gemisches in die Hydrierzone erhalten wird, und der Anteil von Kohlenmonoxid mindestens 0,05 Massen-ppm beträgt — bezogen auf die Masse des Kohlenwasserstoff-Gemisches —,

Überleiten des so gebildeten Reaktionsgemisches in flüssiger Phase über einen fest angeordneten Katalysator mit 0,01 bis 3 Massen-% Palladium, bezogen auf die Masse des Trägers.

2. Verfahren nach Anspruch 1, gekennzeichnet durch Zugeben von Kohlenmonoxid in einer Menge zwischen 0,05 und 20 Massen-ppm.

3. Verfahren nach Anspruch 1, gekennzeichnet durch Überleiten des Reaktionsgemisches über einen Katalysator mit 0,1 bis 2 % Palladium auf inertem Aluminium-Oxid.

4. Verfahren nach den Ansprüchen 1 und 2, gekennzeichnet durch eine Temperatur zwischen 0 °C und 75 °C bei der Hydrierung.

5. Verfahren nach den Ansprüchen 1 bis 3, gekennzeichnet durch ein Monoen haltiges Gemisch von Kohlenwasserstoffen mit drei, vier oder fünf C-Atomen oder mit drei bis fünf C-Atomen.

6. Verfahren nach den Ansprüchen 1 bis 4, gekennzeichnet durch Buten-1 im Kohlenwasserstoff-Gemisch.

## Claims

1. A process for the selective hydrogenation of hydrocarbons having conjugated double bonds, cumulative double or acetylenic triple bonds or combinations thereof in a monoene-containing mixture of hydrocarbons having at least three carbon atoms in the liquid phase on a fixed palladium catalyst on an inert support, which comprises :

adding hydrogen for the hydrocarbon mixture to be hydrogenated, in a finely divided form and in such quantitative ratios that the hydrogen is completely dissolved in the hydrocarbon mixture and a homogeneous liquid phase is obtained before the hydrocarbon mixture enters a hydrogenation zone, said hydrogen being added in an amount from once to twice the stoichiometric quantity for effecting the hydrogenation of the polyunsaturated and acetylenic compounds to the corresponding monoenes ;

adding carbon monoxide to the hydrocarbon mixture to be hydrogenated, in a finely divided form, wherein the carbon monoxide is completely dissolved in the hydrocarbon mixture and a homogeneous liquid phase is obtained before the hydrocarbon mixture enters the hydrogenation zone, and wherein the proportion of carbon monoxide amounts to at least 0,05 ppm by mass based on the mass of the hydrocarbon mixture ; and

passing the resulting reaction mixture in the liquid phase over a fixed catalyst containing 0,01 to 3 % by mass of palladium, based on the mass of the support.

2. A process according to claim 1, wherein carbon monoxide is added in an amount of between 0,05 and 20 ppm by mass of the hydrocarbon mixture.

3. A process according to claim 1, wherein the reaction mixture is passed over a catalyst containing 0,1-2 % by mass of palladium on an inert aluminum oxide.

4. A process according to claims 1 and 2, wherein the reaction conditions include a temperature between 0 °C and 75 °C during hydrogenation.

5. A process according to claims 1 to 3, wherein said monoene-containing mixture of hydrocarbons is a mixture containing a monoene having three, four, or five carbon atoms, or a mixture containing monoenes with three to five carbon atoms.

6. A process according to claims 1 to 4, wherein the monoene in said monoene-containing mixtures is butene-1.

## Revendications

1. Procédé pour l'hydrogénation sélective d'hydrocarbures à doubles liaisons conjuguées et/ou cumulées et/ou à triples liaisons acétyléniques, dans des mélanges d'hydrocarbures à trois atomes de carbone au moins contenant du monoène, en phase liquide, sur des catalyseurs au palladium sur support inerte disposés de façon fixe, caractérisé par

l'addition d'hydrogène au mélange d'hydrocarbures à hydrogéner sous forme finement divisée et dans des rapports quantitatifs tels qu'on obtienne toujours une phase liquide homogène avant l'entrée du mélange d'hydrocarbures dans la zone d'hydrogénation et qui correspondent à une à deux fois la quantité stœchiométrique pour l'hydrogénation des composés poly-insaturés, ainsi que des composés acétyléniques, en monoènes correspondants,

l'addition de monoxyde de carbone au mélange d'hydrocarbures à hydrogéner sous forme finement divisée, une phase liquide homogène étant toujours obtenue avant l'entrée du mélange d'hydrocarbures dans la zone d'hydrogénation et la proportion de monoxyde de carbone étant d'au moins 0,05 ppm en masse, relativement à la masse du mélange d'hydrocarbures,

**0 081 041**

le passage du mélange réactionnel ainsi formé, en phase liquide, sur un catalyseur disposé de façon fixe, contenant de 0,01 à 3 % en masse de palladium, relativement à la masse du support.

2. Procédé selon la revendication 1, caractérisé par l'addition de monoxyde de carbone dans une quantité comprise entre 0,05 et 20 ppm en masse.

3. Procédé selon la revendication 1, caractérisé par le passage du mélange réactionnel sur un catalyseur renfermant de 0,1 à 2 % de palladium sur oxyde d'aluminium inerte.

4. Procédé selon les revendications 1 et 2, caractérisé par une température comprise entre 0 °C et 75 °C lors de l'hydrogénation.

5. Procédé selon les revendications 1 à 3, caractérisé par un mélange d'hydrocarbures à 3, 4 ou 5 atomes de carbone ou à 3-5 atomes de carbone, contenant du monoène.

6. Procédé selon les revendications 1 à 4, caractérisé par du butène-(1) dans le mélange d'hydrocarbures.